(19) Europäisches Patentamt
European Patent Office
Office européen des brevets

(11) Publication number: 0 120 304 A2

(12) EUROPEAN PATENT APPLICATION

(21) Application number: 84101918.5

(22) Date of filing: 23.02.84

(51) Int. Cl.[3]: C 07 D 495/04
C 07 D 333/38, A 61 K 31/335
//(C07D495/04, 333/00, 313/00)

(30) Priority: 28.02.83 US 470684

(43) Date of publication of application: 03.10.84 Bulletin 84/40

(84) Designated Contracting States: AT BE CH DE FR GB IT LI NL

(71) Applicant: HOECHST-ROUSSEL PHARMACEUTICALS INCORPORATED
Route 202-206 North
Somerville New Jersey 08876(US)

(72) Inventor: Martin, Lawrence Leo
R.D. 3, Box 81 Concord Road
Labanon New Jersey 08833(US)

(72) Inventor: Setescak, Linda Louise
102 Kimberly Road
Somerville New Jersey 08876(US)

(74) Representative: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) Substituted 4,10-dihydro-10-oxothieno benzoxepins and intermediates thereof, a process for their preparation and their use as medicaments.

(57) The present invention relates to substitued 4,10-dihydro-10-oxothieno-benzoxepins of the formula

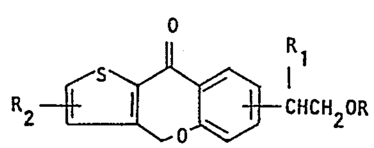

where R is hydrogen or loweralkylcarbonyl; and $R^1$ and $R^2$ are each independently hydrogen or loweralkyl, which are useful as anti-inflammatory and analgetic agents, a process for their preparation and intermediate compounds therefor. Said compounds exhibit an advantageous prolonged duration of action and low gastric irritation properties.

EP 0 120 304 A2

This invention relates to novel 4,10-dihydro-10-oxothienobenzoxepins of the formula

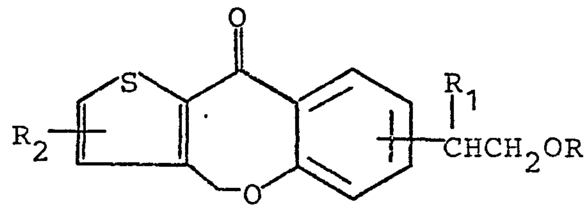

(I)

where R is hydrogen or loweralkylcarbonyl; $R_1$ and $R_2$ are each independently hydrogen or loweralkyl; to intermediate compounds therefor; and to methods of synthesizing the foregoing compounds.

To the best of our knowledge the compounds of the present invention have not been described or suggested.

One group of compounds of the present invention are compounds of the general formula I recited above. Particularly preferred compounds are those of compounds I where $R_2$ is hydrogen and $R_1$ is hydrogen or loweralkyl, particularly methyl.

In the above definitions and as used hereinafter, the term "lower" means the group it is describing contains 1 to 6 carbon atoms preferably 1-4 carbon atoms. The term "alkyl" refers to a straight or branched chain hydrocarbon containing no unsaturation e.g. methyl, ethyl, n-propyl, isopropyl, tertiary-butyl, etc. The term "halogen" refers to a member of the family consisting of fluorine, chlorine, bromine and iodine.

The compounds of the present invention are prepared in the following manner. The groups R, $R_1$ and $R_2$ are as defined above unless indicated otherwise.

Method A

A compound of the formula

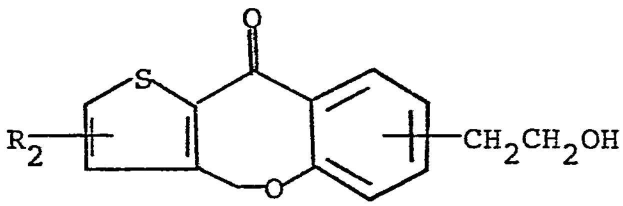

(Ia)

is obtained by reducing a corresponding acid compound of the formula

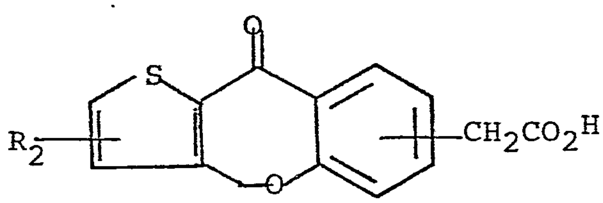

(II)

which is prepared generally according to the manner described in U.S. Patent 4,025,640 (corresponding to German Patent No. 2,637,110), such as for example according to the manner of Example 1 thereof. Said reduction is conducted, for instance, by adding a solution of borane-tetrahydrofuran ($BH_3$/THF) complex to a cooled solution of compound II in dry THF and continuing the reaction at ambient temperature.

Method B

A compound of the formula

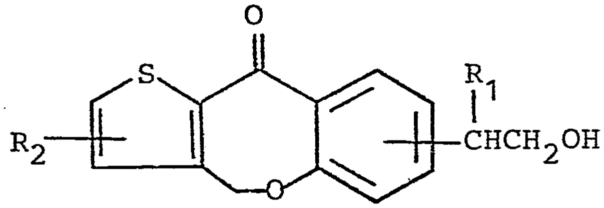

(Ib)

where $R_1$ is hydrogen or loweralkyl, and $R_2$ is hydrogen or loweralkyl is prepared by the cyclization of compound X

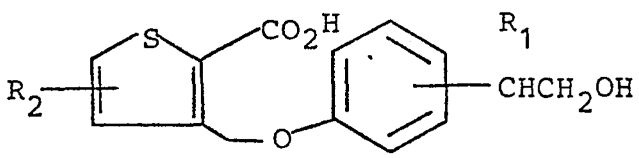

(X)

Said cyclization is conducted in a usual manner by treatment with a dehydrating agent, for instance, by refluxing a solution comprising compound X, trifluoroacetic anhydride and a suitable solvent such as methylene chloride. After a suitable reaction period such as six hours, excess anhydride is decomposed by the addition of 5% HCl, for instance. The resulting trifluoroacetyl ester of Ib is hydrolyzed by conventional methods, o.g. by refluxing with 5 % HCl in a suitable solvent such as acetone, for instance.

The starting compound of the formula X where $R_1$ is loweralkyl is prepared according to the following Scheme I

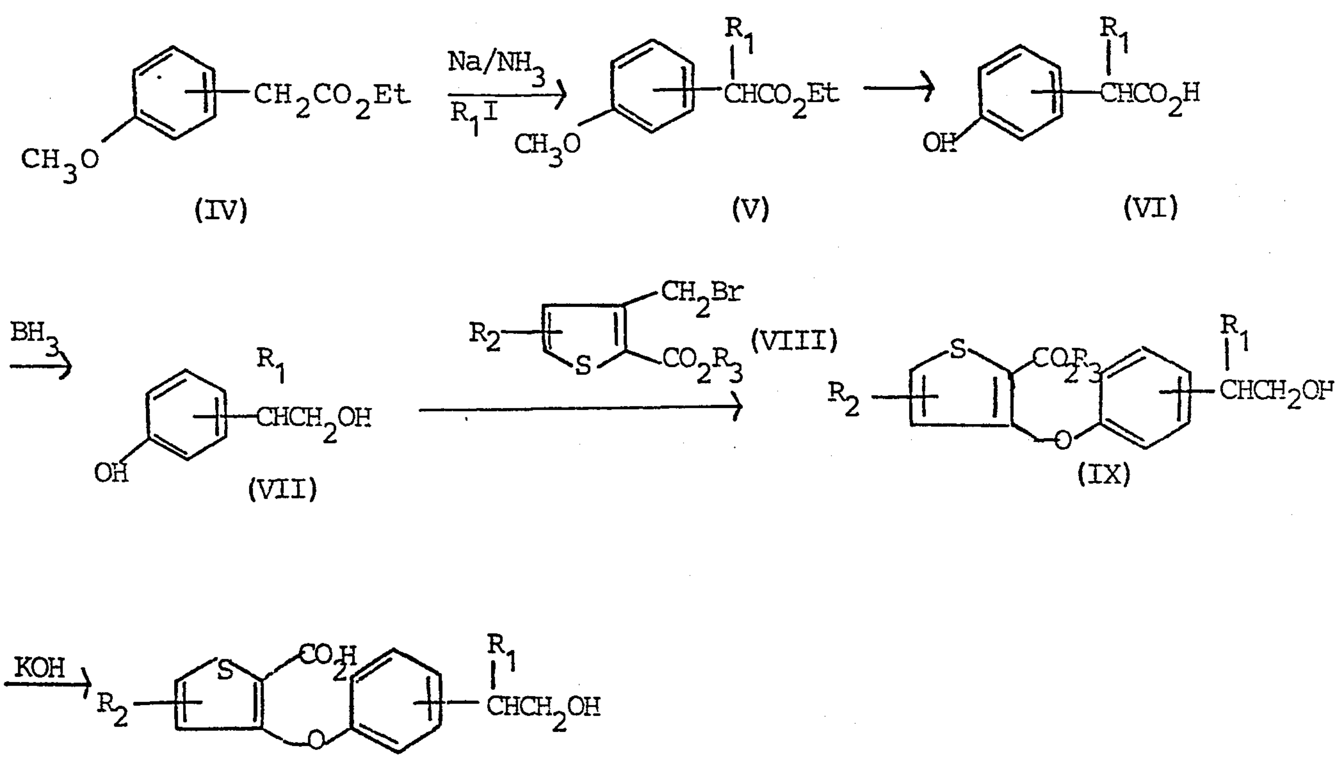


(IV) (V) (VI) (VII) (IX) (X)

(SCHEME )

wherein $R_1$ is loweralkyl, $R_2$ is hydrogen or loweralkyl and $R_3$ is loweralkyl. The respective steps are carried through as follows:

1. The alkylation of compound IV to obtain compound V is conducted, for instance, by preparing a suspension of Na in liquid $NH_3$ containing a small amount of $Fe(NO_3)_3$, adding to the suspension a solution of compound IV in a suitable solvent such as ether, and then adding to the mixture a loweralkyl iodide, $R_1I$.

2.The hydrolysis of compound V to obtain compound VI is conducted, for instance, by adding pyridine hydrochloride to compound V and conducting the reaction at an elevated temperature such as 210°C.

3. The reduction of compound VI to obtain compound VII is conducted, for instance, by adding a $BH_3$/THF solution to a cooled solution of compound VI in dry THF and continuing the reaction at ambient temperature.

4. The condensation reaction between compound VII and compound VIII to obtain compound IX is conducted, for instance, in the presence of potassium carbonate and potassium iodide in a suitable solvent such as 2-butanone under a reflux condition. A preferred example of the group $R_3$ in compound VIII is methyl.

5. The hydrolysis of compound IX to obtain compound X is conducted, for instance, by refluxing compound IX in a solution comprising KOH, water and ethanol, the major component being ethanol.

When $R_1$ is methyl in the above-described reaction sequence (SCHEMA I), one can use an alternative route for the synthesis of compound VI as shown below.

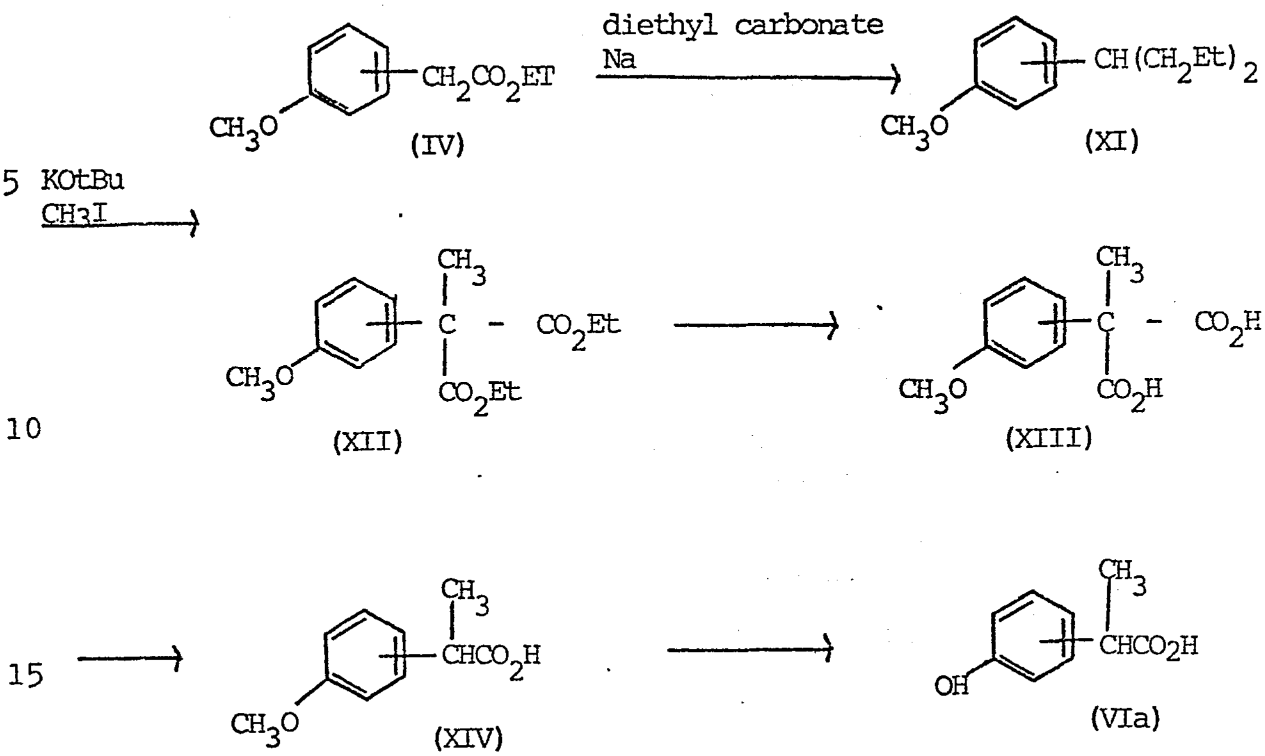


(IV) (XI) (XII) (XIII) (XIV) (VIa)

(SCHEME II)

(i) The conversion of compound IV to compound XI is conducted by reacting compound IV with diethyl carbonate in the presence of Na metal at an elevated temperature such as about 100°C.

(ii) The methylation of compound XI to obtain compound XII is conducted by reacting compound XI with methyl halide such as methyl iodide in the presence of a base such as potassium tertiary butoxide at a moderate temperature such as ambient temperature or lower.

(iii) The hydrolysis of compound XII to afford compound XIII is conducted, for instance, by refluxing compound XII in ethanol containing KOH and a small amount of water.

(iv) The conversion of compound XIII to compound XIV is conducted, for instance, by heating compound XIII at an

elevated temperature such as about 184°C.

(v) The hydrolysis of compound XIV to afford compound VIa is conducted in the same manner as reaction (2) of STEP B described above.

A compound of the formula I where R is loweralkylcarbonyl is obtained by esterifying a compound of the formula

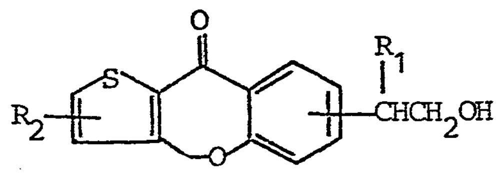

(Ic)

where $R_1$ is hydrogen or loweralkyl, and $R_2$ is hydrogen or loweralkyl, which is obtained as indicated above, by using conventional methods for instance using an acid anhydride of the formula ROR where R is loweralkylcarbonyl. Said esterification is conducted, for instance, in pyridine at an elevated temperature.

All other starting materials shown above are either known compounds or easily prepared by routine methods known in the art from readily available materials.

The compounds of the present invention are useful as antiinflammatory agents due to their ability to suppress inflammation in mammals. The activity of the compounds is demonstrated in the carrageenin induced rat paw edema anti-inflammatory assay (Proc. Soc. Exptl. Biol. Med., III 544 (1962), J. Pharmacol. Exp., <u>141</u> (1963)). Table 1 shows a result of the anti-inflammatory test for some of the compounds of this invention.

TABLE I

Anti-INFLAMMATORY ASSAY

| | $ED_{50}$ (mg/kg; 120 min.) | TIME RESPONSE (20 g/kg) % Inhib. | Pretreat Time (hr.) |
|---|---|---|---|
| (4,10-dihydro-10-oxothieno[3,2-c][1] benzoxepin-8-yl) ethanol | 34.2 | 50 | 1 |
| | | 62,34 | 2 |
| | | 37 | 3 |
| | | 52 | 4 |
| | | 65 | 5 |
| | | 57; 61 | 6 |
| | | 42 | 18 |
| | | 30 | 24 |
| 2-(4,10-dihydro-10-oxothienol[3,2-c][1]-benzoxepin-8-yl)ethyl acetate | 11.4 | | |
| (±)-2-(4,10-dihydro-10-oxothienol[3,2-c][1]-benzoxepin-8-yl)propanol | 4.3 | 25 | 1 |
| | | 43 | 2 |
| | | 50 | 4 |

The compounds of the invention compare favorably with the well known anti-inflammatory compound indomethacin, which, in a similar test, exhibited an anti-inflammatory $ED_{50}$=6 mg/kg, orally.

Compounds of the present invention are also useful as analgesic agents due to their ability to alleviate pain in mammals. The activity of the compounds is demonstrated in the 2-phenyl-1,4-benzoquinone-induced writhing test in mice, a standard assay for analgesia (Proc. Soc. Exptl. Biol. Med., 95, 729 (1957)). Table 2 shows a result of the test of the analgesic activities of some of the compounds of this invention.

TABLE 2

ANALGESIC ACTIVITY
(Phenylquinone Writhing)

| | $ED_{50}$ (mg/kg; p.o.) |
|---|---|
| (4,10-dihydro-10-oxothieno-[3,2-c][1]benzoxepin-8-yl)ethanol | 19.7 |
| (±)-2-(4,10-dihydro-10-oxothieno-[3,2-c][1]benzoxepin-8-yl)propanol | 4.3 |

The compounds of the invention compare favorably with the well known analgesic compound ibuprofen, which, in a similar test exhibited an analgesic $ED_{50}$ = 10.4 mg/kg, orally.

Several of the compounds of this invention were evaluated for their ability to induce gastric irritation (ulcerogenic activity). The method utilized to evaluate the ulcerogenic activity is as follows:
(See Journal of Medicinal Chemistry, 1977, Vol. 20, No. 1, pp.66-70); Groups of eight male Wistar rats weighing 150-175 grams were fasted 48 hours (water ad libitum)prior to administration of the test drug orally (10 ml/kg). Control rats received vehicle only (10 ml/kg). For a time

response, animals were treated with a highly active anti-inflammatory dose of the test drug and then sacrificed at 3, 5 and 7 hour postdrug. Stomachs and intestines were removed and examined for the presence of lesions. The presence of single or multiple lesions (erosion, ulcer, or perforation) was considered an ulcerogenic effect. A dose response was performed at the peak time using four doses of test drug. The results of the gastric irritation tests are summarized in Table 3. They show low gastric irritation properties of the compounds.

TABLE 3

GASTRIC IRRITATION ACTIVITY

| | $ID_{50}$ (mg/kg; p.o.) |
|---|---|
| (4,10-dihydro-10-oxothieno-[3,2-c][1]benzoxepin-8-yl)ethanol | > 400 |
| 2-(4,10-dihydro-10-oxothieno-[3,2-c][1]benzoxepin-8-yl)ethyl acetate | 175 |
| 2-(4,10-dihydro-10-oxothieno-[3,2-c][1]benzoxepin-8-yl)ethyl propionate | > 100 |
| (±)-2-(4,10-dihydro-10-oxothieno-[3,2-c][1]benzoxepin-8-yl)propanol | 24.2 |

Effective quantities of the compounds of the invention may be administered to a patient by any of the various methods, for example, orally as in capsules or tablets, parenterally in the form of sterile solutions or suspensions, and in some cases intravenously in the form of sterile solutions. The free acid final products, while effective themselves, may be formulated and administered

in the form of their pharmaceutically acceptable addition salts for purposes of stability, convenience of crystallization, increased solubility and the like.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5 % of active compound, but may be varied depending upon the particular form and may conveniently be between 4 % to about 70 % of the weight of the unit. The amount of active compound in such compositions is such that a suitable dosage will be obtained. Preferred compositions and preparations according to the present invention are prepared so that an oral dosage unit form contains between 1.0-300 milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, cornstarch and the like; a lubricant such as magnesium stearate; a glidant such as colloidal silicon dioxide; and a sweetening agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus tablets or pills may be coated

with sugar, shellac, or other enteric coating agents. A syrup may contain, in addition to the active compounds, sucrose as a sweetening agent and certain preservatives, dyes, colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1 % of active compound, but may be varied to be between 0.5 and about 30 % of the weight thereof. The amount of active compound in such compositions is such that a suitable dosage will be obtained. Preferred compositions and preparations acording to the present invention are prepared so that a pareneral dosage unit contains between 0.5 to 100 milligrams of active compound.

The solutions or suspension may also include the following components: a sterile diluent such as water for injection, saline solution, fixed oils,polyethylene glycols, glycerine, propylene glycol or other synthetic solvents; antibacerial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in disposable syringes or multiple doze vials made of glass or plastic.

Examples of the compounds of this invention include:
(4,10-dihydro-10-oxothieno[3,2-c][1]benzoxepin-8-yl)ethanol;
2-(4,10-dihydro-10-oxothieno[3,2-c][1]benzoxepin-8-yl)ethyl acetate;
2-(4,10-dihydro-10-oxothieno[3,2-c][1]benzoxepin-8-yl)ethyl propionate;

(±)-3-[4-(1-hydroxyprop-2-yl)phenoxy]methyl-2-thiophenecarboxylic acid ;
and (±)-2-(4,10-dihydro-10-oxothieno[3,2-c][1]benzoxepin-8-yl)propanol

The following examples are given for illustrative purposes and are not to be considered as limiting the invention disclosed herein. All temperatures are given in degrees Celcius.

EXAMPLE 1

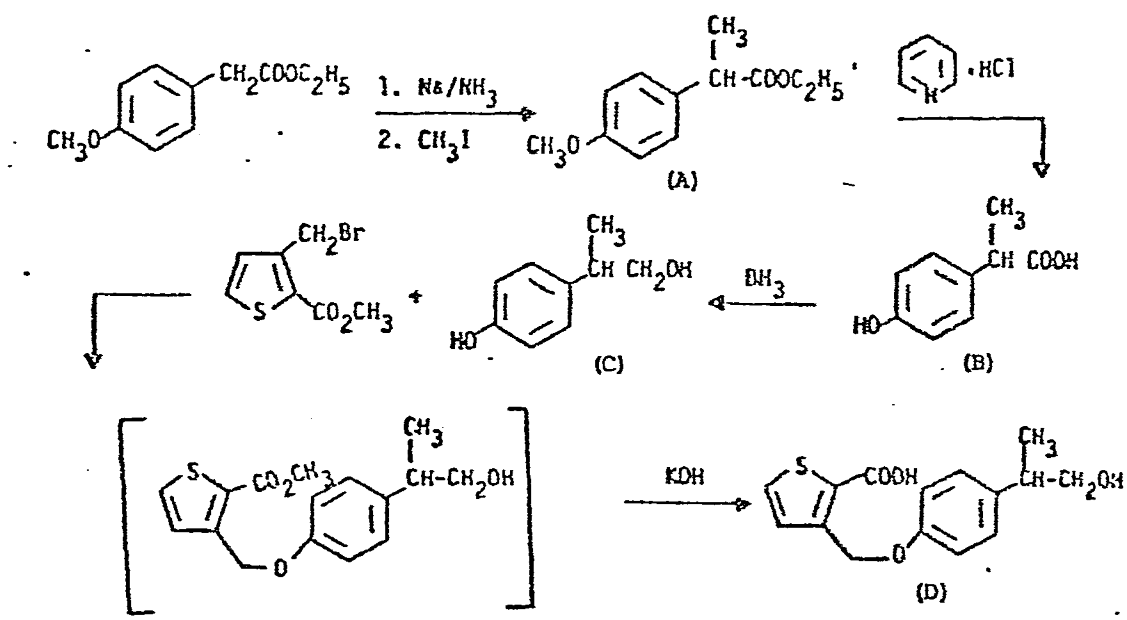


(±)-3-[4-(1-Hydroxyprop-2-yl)phenoxy]methyl-2-thiophenecarboxylic acid

This synthesis required a number of steps which are described in detail. Synthesis of compound A by $NaNH_2/CH_3I$ alkylation of ethyl 4-methoxyphenylacetate

afforded a mixture of the propionate and starting acetate which was converted to crude compound C. HPLC separation afforded compound C as a pure material which was employed for the synthesis of compound D. An alternative preparation of (±)-2-(4-methoxyphenyl)propionic acid from ethyl 4-methoxyphenylacetate via diethyl 4-methoxyphenylmalonate is also described and cleavage of the methyl ether with pyridine hydrochloride as described herein gives compound B.

Ethyl(±)-2-(4-methoxyphenyl)propionate[1] (A)

To 500-600 ml of $NH_3$, 0.25 g of $Fe(NO_3)_3$ was added followed by 0.5 g of Na. The mixture was stirred until the deep blue color dissipated to afford a gray suspension. To this mixture 11.49 g (0.5 m) of Na was added. The blue color also dissipated to afford a gray suspension over approximately 30-35 minutes. A solution of 97.11 g (0.5 m) of ethyl p-methoxyphenylacetate in 25 ml of ether was added dropwise. The mixture was stirred for 15 minutes followed by the dropwise (nonself-compensating dropping funnel) addition of 70.97 g (0.5 m) of $CH_3I$. Additional $CH_3I$ was added until the deep blue color dissipated to give a yellowish color. The mixture was stirred for 1 hour and quenched with a slight excess of 1 molar equivalent of $NH_4Cl$. The ammonia was allowed to evaporate to dryness with stirring. The residue was partitioned between water and ether. The ether extract was washed with 5 % $NaHCO_3$ and then with saturated NaCl solution, dried ($Na_2SO_4$), filtered and evaporatd to give 89.48 g of product as an oil. By GC analysis the crude product was composed of 88.1 % of the propionated and 8.8 % of the acetate starting material.

---

[1] W.G. Kenyon, E.M. Kaiser and C.R. Hauser, J. Org. Chem. 30, 2937 (1965).

(±)-2-(4-Hydroxyphenyl)propionic acid (B)

A mixture of 89 g (0.427 m) of ethyl(±)-2-(4-methoxyphenyl)-propionate and 450 g of pyridine hydrochloride was placed in an oil bath at 210° for 3 hours. The mixture was cooled and partitioned between water and ether. The ether extract was washed several times with water. When the ether extract was dried and evaporated only 5.9 g of product was isolated. The aqueous layer was concentrated, sodium chloride was added to saturation and the solution was extracted five times with ether to give 36.13 g of product. The aqueous layer was then subjected to continuous extraction with ether overnight to give an additional 24 g of product. The total yield was 64.49 g. The spectral data were consistent with the structure.

(±)-2-(4-Hydroxyphenyl)propanol (C)

To a cooled solution (-10°-0°) of 24 g (0.144 m) of compound B in 200 ml of sieve dried THF, 235 ml of $BH_3$ (0.97 m $BH_3$/THF) was added dropwise. The mixture was stirred overnight at ambient temperature. The reaction mixture was cooled and excess $BH_3$ was decomposed by the addition of excess methanol. The solvents were removed in vacuo. The residue partitioned between $CH_2Cl_2$ and $NaHCO_3$. The organic extract was washed with water until neutral, dried ($Na_2SO_4$), filtered and evaporated. Only a small quantity of product was obtained. The aqueous layer was then continuously extracted with $CH_2Cl_2$. The resultant organic extract was dried ($Na_2SO_4$), filtered and evaporated to give 14 g of alcohol, (HPLC analysis: 84.5 % propanol derivative, 12.5 % ethanol derivative). The mixture was separated on a liquid chromatography system (Silica gel; Solvent system: EtOAc: hexane, 1:1, alcohol mixture applied as a solution in 130 ml EtOAc and 130 ml $CH_2Cl_2$) and 8 g of pure compound C was obtained.

(±)-3-(4-(1-hydroxyprop-2-yl)phenoxymethyl-2-thiophenecarboxylic acid

A mixture of 8 g (0.0526 m) of compound C, 12 g (0.0526 m) of 3-(α-bromomethyl)-2-carbomethoxythiophene, 29 g of $K_2CO_3$ (0.021 m), 0.5 g of KI and 200 ml of 2-butanone was refluxed overnight. The mixture was filtered and the filtrate was evaporated. The residue was dissolved in ether and was washed with 5 % NaOH and then with water until neutral. The ether extract was dried ($Na_2SO_4$), filtered and evaporated to an oil composed chiefly of the trifluoroacetyl ester. The ester was hydrolyzed by refluxing 15.5 g of the oil in a solution of 28.1 g of KOH in 28 ml of water and 100 ml of 95 % ethanol for 5.5 hours. The ethanol was evaporated and the residual liquid was poured over ice and acidified with cold concentrated HCl. An oil formed which gradually solidified. The solid was filtered, washed with water and dried in vacuo. The material was recrystallized from acetonitrile to give 11.72 g (80 %) of product, m.p. 123-125°C.

ANALYSIS:

| | | |
|---|---|---|
| Calculated for $C_{15}H_{16}O_4S$: | 61.63 % C | 5.52 % H |
| Found: | 61.61 % C | 5.56 % H |

AN ALTERNATIVE SYNTHETIC ROUTE

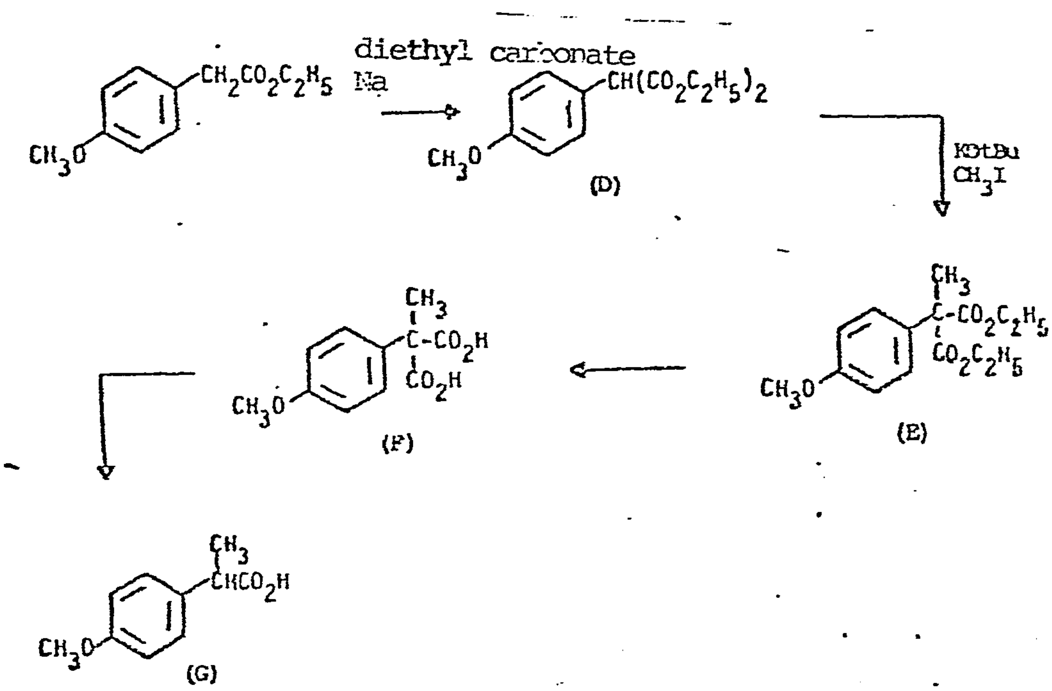


(±)-2-(4-methoxyphenyl)propionic acid

Diethyl 4-methoxyphenylmalonate (D)

A stirred mixture of ethyl 4-methoxyphenylpropionate (102.11 g, 0.526 mol), diethylcarbonate (750 ml) and sodium metal (13.92 g, 0.605 g-atom, 15 % excess) was heated to approximately 98°C ($N_2$ atmosphere). An exothermic reaction initiated and the heating mantle was removed. When the reaction subsided, the stirred solution was heated under reflux for one hour. Diethylcarbonate was then removed from the hot turbid mixture to afford an oil which solidified. The cooled residue was then treated with ice cold water (500 ml) and extracted with ether (approximately 1000 ml employed, all solid dissolved). The organic phase was washed with water (300 ml) and saturated sodium chloride solution. The dried

($Na_2SO_4$) organic phase was filtered and concentrated to an oil (192.8 g) which was distilled to afford 108.9 g (77.8 %) of diethyl 4-methoxyphenylmalonate, b.p. 135°-140°C (0.3 mm Hg). A GC analysis indicated that the material was 98.17 % pure and contained 0.36 % of an impurity with the same retention time as ethyl 4-methoxyphenylacetate. This synthesis is based on the preparation of this compound as described by G. Zuilichousky and U. Fotadar, Org. Prep. Proc. Int., 6, 5-9 (1974). Spectral data were consistent with the structure.

Diethyl (4-methoxyphenyl)methylmalonate (E)

A solution of compound D(88.47 g, 0.332 mol) and sieve dried tetrahydrofuran (140 ml) was added dropwise over 14 minutes with cooling (14°C) to a stirred solution of potassium t-butoxide (42.87 g, 0.382 mol) and tetrahydrofuran (330 ml). The solution ws chilled to 8°C and a solution of iodomethane (54.22 g, 0.382 mol, 15 % excess) and tetrahydrofuran (100 ml) was added over 4 minutes (internal temperature reached 18°C). Near the end of the addition, a precipitate separated. The mixture was stirred for 2 hours with cooling and 1 hour at ambient temperature. The mixture was filtered and the filter cake was washed with a little ether. The combined filtrate was washed once with water and 10 % NaOH solution and then was washed twice with water. The dried ($Na_2SO_4$), filtered organic phase was concentrated to an oil which was vacuum distilled to afford 75.04 g (80.6 %) of product, b.p. 125-130°C (0.12 mm Hg). The product was reported by P.J. Scheuer and S.G. Cohen, J.Am. Chem. Soc., 80, 4933 (1958) and by J.T. Pinley and B.A. Rowe, Tet. Lett., 21 965 (1980); however, the above synthesis was not employed by either group. Spectral data were consistent with the structure.

ANALYSIS

| | | |
|---|---|---|
| Calculated for $C_{11}H_{12}O_5$: | 64.27 % C | 7.19 % H |
| Found: | 64.59 % C | 7.35 % H |

(4-Methoxyphenyl)methylmalonic acid (F)

A stirred solution of KOH (150.37 g, 2.68 mol), 95 % methanol (800 ml) and 75.04 g (0.268 mol) of compound E was refluxed for 2 hours, during which a precipitate separated. The suspension was diluted with water to afford a solution which was concentrated on a rotary evaporator to remove the ethanol. The residual solution was treated with 1 l of crushed ice and acidified with concentrated HCl (250 ml). The mixture was extracted with ether (3 x 400 ml) and the combined, dried ($Na_2SO_4$) organic phase was filtered and concentrated to afford a pasty solid. Residual water was removed by azeotropic distillation with toluene. The residue was suspended in toluene, isolated by vacuum filtration, washed with toluene and hexane and dried in vacuo at 40°C to give the crude product (57.15 g). Recrystallization from acetonitrile (200 ml) gave 48.69 g (81.03 %) of crystals, m.p. 158-159°C (gas evolution). The compound was previously reported by W.M. Lauer and L.I. Hansen, J. Am.Chem. Soc., 61, 3039 (1939), m.p. 149.5-150°C. Spectral data were consistent with the structure.

ANALYSIS:

| | | |
|---|---|---|
| Calculated for $C_{11}H_{12}O_5$: | 58.92 % C | 5.40 % H |
| Found: | 59.00 % C | 5.49 % H |

(±)-2-(4-Methoxyphenyl)propionic acid (G)

Compound F (48.5 g, 0.216 mol) was placed in a round bottom flask equipped with a magnetic stirrer. The flask was immersed in a preheated (184°C) oil bath. Within a few minutes, melting and gas evolution initiated. The residual oil was stirred with heating for 10 minutes after the gas evolution ceased. The oil was cooled and triturated with hexane to afford a suspension of crystalline material.

The material was isolated by vacuum filtration, washed with hexane and dried in vacuo at ambient temperature to give 35.39 g (90.9 %) of crystals, mp. 54-56.5°C. Spectral data were consistent with the structure. The compound was previously reported by W.G. Kenyon, E.M. Kaiser and C.R. Hauser, J. Org. Chem., 30, 2937 (1965), m.p. 61-62.5°C (hexane) and by V.N. Gupta and T.R. Seshadri, Proc. Indian Acad. Sci., 44A, 223 (1956), m.p. 55-57°C.

Example 2

(±)-2-(4,10-Dihydro-10-oxothieno[3,2-c][1]benzoxepin-8-yl)propanol

To a solution of 5.5 g (0.0188 m) of (±)-3-[4-(1-hydroxyprop-2-yl)phenoxy]methyl-2-thiophenecarboxylic acid in 50 ml of $CH_2Cl_2$, 9.88 g of trifluoroacetic anhydride was added dropwise. The mixture was refluxed for six hours. Excess anhydride was decomposed by the addition of 5 % HCl. The organic layer was separated, washed with water until neutral, dried ($Na_2SO_4$), filtered and evaporated to give an oil.

A mixture of the oil, 100 ml of 5 % HCl and 200 ml of acetone was refluxed for 2 hours. The acetone was evaporated and the residual layer was extracted with ether. The ether extract was washed with 5 % $NaHCO_3$, and then with water until neutral, dried ($Na_2SO_4$), filtered and evaporated to an oil. The oil was triturated with diisopropyl ether to give 3.89 g (76 %) of solid, m.p. 66.5-68°C.

ANALYSIS:

| | | |
|---|---|---|
| Calculated for $C_{15}H_{14}O_3S$: | 65.67 % C | 5.14 % H |
| Found: | 65.86 % C | 5.11 % H |

EXAMPLE 3

(4,10-Dihydro-10-oxothieno[3,2-c][1]benzoxepin-8-yl) ethanol

To a solution of 10 g (0.036 m) of 4,10-dihydro-10-oxothieno-[3,2-c][1]benzoxepin-8-acetic acid in 50 ml of anhydrous THF (temperature -20°), 72 ml of $BH_3$/THF (0.072 m) was added dropwise. After the addition the mixture was allowed to warm to ambient temperature and was stirred overnight at ambient temperature. The reaction mixture was treated with MeOH. The solvents were evaporated and the residue was partitioned between water and $CH_2Cl_2$. The $CH_2Cl_2$ extract was washed with $NaHCO_3$, dried over $Na_2SO_4$, filtered and evaporated to afford a crude oil. Trituration of the oil with hot hexane gave 3.9 g (40 %) of the product as an oil.

ANALYSIS:

| | | |
|---|---|---|
| Calculated for $C_{14}H_{12}SO_3$: | 64.59 % C | 4.65 % H |
| Found: | 64.62 % C | 4.84 % H |

EXAMPLE 4

2-(4,10-Dihydro-10-oxothieno[3,2-c][1]benzoxepin-8-yl) ethyl acetate

To a solution of 5 g (0.019 m) of (4,10-dihydro-10-oxothieno[3,2-c][1]benzoxepin-8-yl)ethanol in 25 ml of pyridine, 5.92 g (0.058 m) of acetic anhydride was added dropwise. The mixture was warmed (steam bath) for 5 minutes, poured into 200 ml of water and stirred overnight. The mixture was extracted with $CH_2Cl_2$. The $CH_2Cl_2$ extract was washed with 5 % HCl and then with 10 % NaOH. The extract was further washed with water, dried ($Na_2SO_4$), filtered and evaporated to give 4.9 g (86 %) of the product as an oil.

ANALYSIS:

| | | |
|---|---|---|
| Calculated for $C_{16}H_{14}SO_4$: | 63.56 % C | 4.67 % H |
| Found: | 63.84 % C | 4.67 % H |

EXAMPLE 5

2-(4,10-Dihydro-10-oxothieno[3,2-c][1]benzoxepin-8-yl)-ethyl propionate

To a solution of 5 g (0.019 m) of (4,10-dihydro-10-oxothieno[3,2-c][1]benzoxepin-8-yl)ethanol in 25 ml of pyridine, 7.55 g (0.058 m) of propionic anhydride was added dropwise. The mixture was warmed on a steam bath for 5 minutes with the exclusion of moisture. The reaction was quenched by decanting the mixture into 150 ml of ice water. The mixture was extracted with an ether/ethyl acetate mixture. The extract was washed twice with 5 % HCl and twice with 5 % $NaHCO_3$, dried ($Na_2SO_4$), filtered and evaporated to give 4.7 g (79 %) of the product as an oil.

ANALYSIS:

| | | |
|---|---|---|
| Calculated for $C_{17}H_{16}O_4S$: | 64.54 % C | 5.10 % H |
| Found: | 64.42 % C | 5.08 % H |

Claims:

1. A compound having the formula I

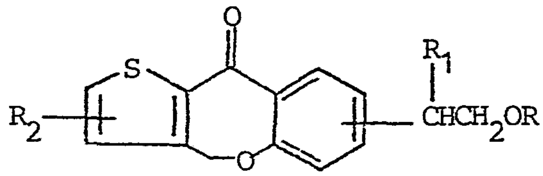

(I)

where R is hydrogen or loweralkylcarbonyl; $R_1$ is hydrogen or loweralkyl; and $R_2$ is hydrogen or loweralkyl.

2. A compound of the formlua I as claimed in claim 1 where $R_2$ is hydrogen and $R_1$ is hydrogen or loweralkyl.

3. A compound of the formula I as claimed in claim 2 where $R_1$ is hydrogen or methyl.

4. A compound of the formula I as claimed in claim 3 where R is hydrogen or acetyl.

5. The compound as claimed in claim 1 which is 2-(4,10-dihydro-10-oxothieno[3,2-c][1]benzoxepin-8-yl)ethanol.

6. The compound as claimed in claim 1, which is (±)-2-(4,10-dihydro-10-oxothieno[3,2-c][1]benzoxepin-8-yl)-propanol.

7. The compound as claimed in claim 1 which is 2-(4,10-dihydro-10-oxothieno[3,2-c][1]benzoxepin-8-yl)-ethyl acetate.

8. A pharmaceutical composition comprising as active ingredient a compound of the formula I as claimed in claim 1 in association with a pharmaceutically acceptable carrier and/or stabilizer.

9. A compound of the formula I as claimed in claim 1 for use as a medicament.

10. A process of preparing a compound of the formula I

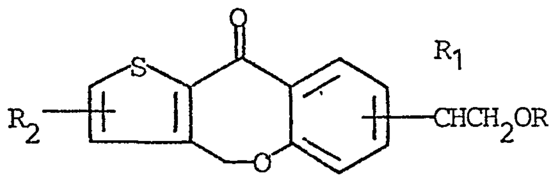

(I)

where R is hydrogen or loweralkylcarbonyl, $R_1$ is hydrogen or loweralkyl and $R_2$ is hydrogen or loweralkyl, which comprises

a) reducing a compound of the formula II

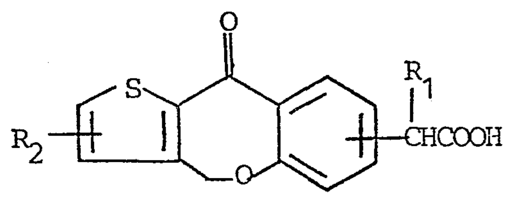

(II)

wherein $R_1$ and $R_2$ are as defined above, to yield a compound of the formula I, wherein R is hydrogen,

b) or cyclizing a compound of the formula X

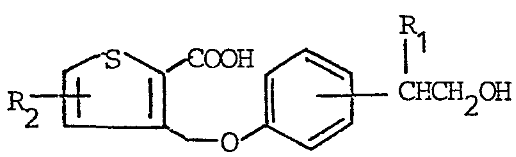

(X)

wherein $R_1$ and $R_2$ are as defined above to yield a compound of the formula I wherein R is hydrogen, and

c) optionally esterifying a compound of the formula I wherein R is hydrogen, to form a compound of the formula I wherein R is loweralkyl carbonyl.

11. A compound of the formlua X

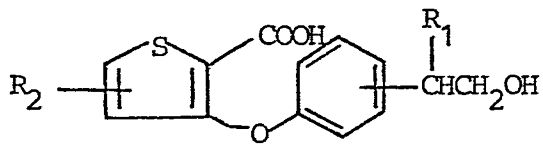

(X)

wherein $R_1$ is hydrogen or loweralkyl and $R_2$ is hydrogen or loweralkyl.

12. A process of preparing a compound of the formula X

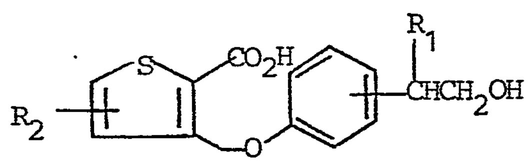

(X)

where $R_1$ is hydrogen or loweralkyl and $R_2$ is hydrogen or loweralkyl, which comprises the hydrolysis of a compound of the formula

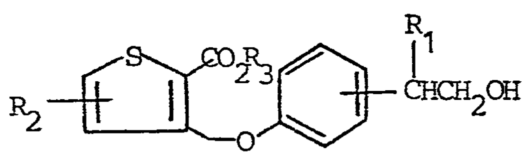

(IX)

where $R_3$ is loweralkyl.

0120304
HOE 83/S 006

Claims for Austria:

1. A process of preparing a compound of the formula I

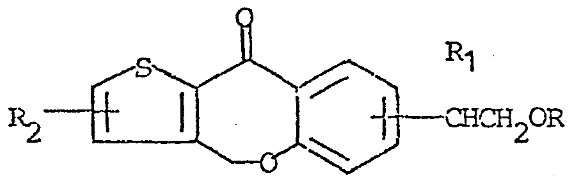

(I)

where R is hydrogen or loweralkylcarbonyl, $R_1$ is hydrogen or loweralkyl and $R_2$ is hydrogen or loweralkyl, which comprises

a) reducing a compound of the formula II

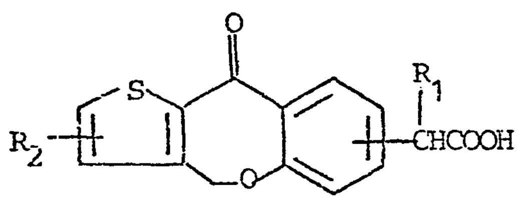

(II)

wherein $R_1$ and $R_2$ are as defined above, to yield a compound of the formula I, wherein R is hydrogen,

b) or cyclizing a compound of the formula X

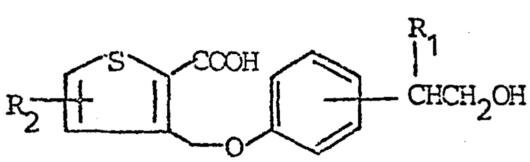

(X)

wherein $R_1$ and $R_2$ are as defined above to yield a compound of the formula I wherein R is hydrogen, and

c) optionally esterifying a compound of the formula I wherein R is hydrogen, to form a compound of the formula I wherein R is loweralkyl carbonyl.

2. A compound of the formlua X

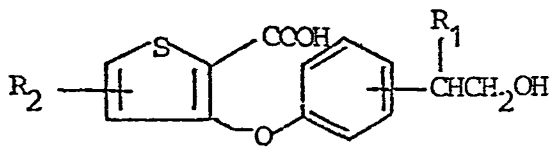

(X)

wherein $R_1$ is hydrogen or loweralkyl and $R_2$ is hydrogen or loweralkyl.

3. A process of preparing a compound of the formula X

(X)

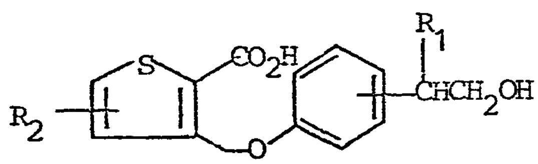

where $R_1$ is hydrogen or loweralkyl and $R_2$ is hydrogen or loweralkyl, which comprises the hydrolysis of a compound of the formula

(IX)

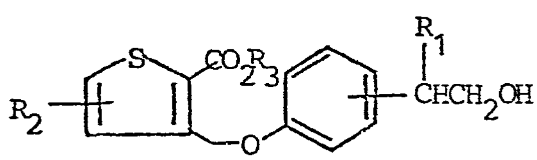

where $R_3$ is loweralkyl.